# EUROPÄISCHE PATENTSCHRIFT

(11) **EP 4 297 567 B1**
(45) Veröffentlichungstag und Bekanntmachung des Hinweises auf die Patenterteilung: **02.07.2025**
(21) Anmeldenummer: 22710339.7
(22) Anmeldetag: 23.02.2022
(51) Int. Cl.: B65G 1/04, A01K 67/368

(54) **TRANSPORTVORRICHTUNG UND -VERFAHREN BEI DER INSEKTENZUCHT**
TRANSPORT DEVICE AND METHOD IN INSECT BREEDING
DISPOSITIF ET MÉTHODE DE TRANSPORT POUR L'ÉLEVAGE D'INSECTES

(30) Priorität: 24.02.2021 EP 21159092
(43) Veröffentlichungstag der Anmeldung: 03.01.2024
(73) Patentinhaber: Illucens GmbH, 48683 Ahaus (DE)
(72) Erfinder: WESSENDORF, Dirk, 48683 Ahaus (DE)
(74) Vertreter: Taruttis, Tilman
(86) Internationale Anmeldenummer: PCT/EP2022/054577
(87) Internationale Veröffentlichungsnummer: WO 2022/180128

(56) Entgegenhaltungen:
- CN-A- 1 135 281
- CN-A- 1 244 341
- CN-A- 104 604 811
- DE-A1- 102011 012 424
- DE-U1- 202004 003 020
- DE-U1- 202004 003 020
- US-A1- 2017 360 014

## Beschreibung

Die Deckung des Proteinbedarfs von Menschen und/oder Tieren kann über eine Bereitstellung von Insekten erfolgen, die insbesondere industriell gezüchtet und produziert werden können. Als Insekt für die industrielle Zucht kommt die schwarze Soldatenfliege (Hermetia illucens) in Betracht, die hierfür gewünschte Eigenschaften eines Insekts kombiniert. Die Larven der schwarzen Soldatenfliege nehmen schnell an Gewicht zu und für das Futter der Larven können biologische Reststoffe verwendet werden.

Im Bereich der Insektenaufzucht und -produktion ist es in der Regel notwendig, Insekten von einem Ort zu einem anderen Ort zu bewegen, um je nach Entwicklungsstadium bzw. Produktionsschritt jeweils unterschiedliche Umgebungen unterschiedliche, insbesondere optimierte, d.h. für das jeweilige Entwicklungsstadium angepasste, Lebensbedingungen zur Verfügung zu stellen. Der im Stand der Technik bekannte Transport von Insekten ist aufwändig. Bisher werden bei der Insektenaufzucht insbesondere die Insektenlarven meist sogar von dem Anwender in einem Behältnis von einem Ort zum anderen Ort getragen oder Behältnisse mit den Insekten mit Hilfe mechanischer Transporteinrichtung verfahren. Bekannte aufwändigere Transport-einrichtungen für Stückgüter oder Futtermittel werden nicht für den Transport lebender Tiere eingesetzt und versprechen vor allem keinen ausreichend schonenden Transport der Insekten.

Der Transport von stückigem und/oder flüssigem Gut, insbesondere Insekten (in unterschiedlichen Entwicklungsphasen, insbesondere als Insektenlarve), Futter und/oder Wasser zu einer Aufzuchtstation bei der Insektenzucht stellt somit eine Herausforderung dar.

Der Stand der Technik nach US 2017/0360014 A1 bezieht sich auf eine autonome Futterlieferplattform, die zum Navigieren durch eine Einrichtung und zur Lieferung von Insektenfutter an mehrere innerhalb der Einrichtung befindliche Insektenhabitate eingerichtet ist. CN 1 244 341 A betriff eine Madenzuchtmaschine mit einem Futterwagen, der auf Schienen läuft. CN 1 135 281 A betrifft ein automatisiertes Gerät zum Sammeln und Kultivieren von Maden. CN 104 604 811 A1 betrifft eine mehrschichtige intermittierende bewegliche Fliegenlarvenzuchteinrichtung. Die Fliegenlarvenzuchteinrichtung umfasst einen Fliegenlarvenabscheider, eine Chargenzuführeinrichtung, eine Zuchteinrichtung, einen Hilfsauflader, ein Sammelförderband und eine Steuervorrichtung.

Aufgabe der Erfindung ist es, eine möglichst effiziente und/oder ressourcensparende Möglichkeit zu schaffen, Insekten, Futter und/oder Wasser, gezielt an mehrere unterschiedliche Orte zu transportieren.

Die Aufgabe wird mit dem Gegenstand der unabhängigen Ansprüche gelöst.

Vorteilhafte Ausgestaltungen der Erfindung sind Gegenstand der Unteransprüche.

Gemäß einem Kerngedanken der Erfindung wird ein Regalbediengerät verwendet, um die Insekten bzw. Insektenlarven, Futter und/oder Wasser bei der Insektenzucht jeweils mehreren Räumen zuzuführen. Mittels des Regalbediengeräts kann ein Austrag bei unterschiedlichen Räumen jeweils an bzw. in einem Raum erfolgen. Der Austrag bzw. das Zuführen erfolgt mittels eines am Kopf des Regalbediengeräts, an dem im Stand der Technik üblicherweise ein Greifer angeordnet ist, vorhandenen Austragkopf.

Unterschiedliche Räume, die der Austragkopf des Regalbediengeräts anfährt bzw. ansteuert können mit den unterschiedlichen "Gütern" bzw. Substanzen beschickt werden. Insbesondere können unterschiedliche "Güter" zu unterschiedlichen Zeitpunkten der Entwicklungsphase einer Insektenlarve zugeführt werden.

Der Begriff "Regalbediengerät" umfasst im Sinne der Beschreibung ein Fahrzeug, welches entlang der Längserstreckung und der Höhe eines Regals verfahren werden kann. In der Regel weist ein Regalbediengerät ein mittels des Fahrzeugs verfahrbares Lastaufnahmemittel auf, mittels dessen eine Ware bzw. eine Last in oder aus dem Regal, insbesondere unter Zuhilfenahme eines Greifers, heraus bewegt werden kann. Das Lastaufnahmemittel des Regalbediengeräts im Sinne der Erfindung weist den Austragkopf auf bzw. trägt den Austragkopf. Mit dem Austragkopf ist mit einer Zuführung verbunden, mittels derer der Austragkopf mit dem jeweiligen Gut, in Form von Insektenlarven, Futter und/oder Wasser, beschickt werden kann.

Sofern vorliegend eine Zuführung von Insekten/Insektenlarven, Futter und/oder Wasser beschrieben ist, so ist hierunter auch eine Kombination der genannten Substanzen zu verstehen, insbesondere ein Futter und Insekten/Insektenlarven-Gemisch. Die Erfindung betrifft aber nicht nur die Zuführung der zuvor genannten "Güter", sondern auch den Transport von jedwedem stückigen oder flüssigen Gut, so dass in einer Ausgestaltung der Erfindung auch ein Transport von stückigem und/oder flüssigem Gut mittels eines Regalbediengeräts die Erfindung für sich tragen kann.

Eine die Erfindung für sich begründende Aufzuchtstation für Insekten, insbesondere in unterschiedlichen Entwicklungsphasen, in der insbesondere das Regalbediengerät verwendet werden kann, kann regalartige Einrichtungen mit einer vorzugsweise klimatisierten Umgebung aufweisen. In Fächern, Schächten, Wannen oder ähnlichem der regalartigen Einrichtung, die vereinfachend als Regal bezeichnet wird, können Insekten aufgezogen werden. Den Regalfächern sind dazu die Insekten, insbesondere Insektenlarven, Futter und/oder Wasser zuführbar. Die Regalfächer können stationär angeordnet sein. Die Regalfächer können Teil eines Bioreaktors sein.

Insbesondere können die Insekten transportiert bzw. bewegt werden, ohne dass Behältnisse, in denen die Insekten aufgenommen sind, bewegt werden. Die Erfindung hat sich in einem Aspekt von dem grundsätzlichen Gedanken gelöst, dass der Transport mittels bewegter Behältnisse mit Insekten, insbesondere Larven, von einem Ort zu einem anderen Ort bewerkstelligt werden muss.

Es lässt sich dadurch eine effiziente, platzsparende und/oder automatisierte Zuführung von Insektenlarven, Futter und/oder Wasser bei der Insektenzucht erreichen.

Gemäß einem zweiten Kerngedanken der Erfindung wird ein Austragkopf geschaffen, der mehrere Auslässe aufweist, die zur wahlweisen Zuführung von Insektenlarven, Futter, Wasser oder anderem Material in einen Regalfach, vorgesehen sind. Der Austragkopf ist verfahrbar, um ein bestimmtes Regalfach anzusteuern. Vorzugsweise sind mehrere Austragsköpfe nebeneinander oder übereinander vorgesehen.

Der Begriff "Raum" umfasst hier eine örtliche Anordnung, die durch eine Struktur vorgegeben sein kann. Die Struktur kann in Form einer regalartigen Einrichtung vorliegen. Der Raum ist ein Regalfach in der regalartigen Einrichtung sein, welches seitlich durch Seitenwände begrenzt sein kann. Die bodenseitige Begrenzung des Regalfachs kann durch den Boden des Regals vorgegeben sein. Die vorderseitige Begrenzung des Regalfachs kann durch einen festen Abschluss, der eine Klappe aufweisen kann, gebildet sein. Die rückseitige Begrenzung des Regalfachs kann einen verschwenkbaren Verschluss aufweisen.

Der Begriff "Raum" kann ein zumindest teilweise umschlossener Raum eines Regals sein. Insoweit bietet ein Regal die Möglichkeit in Spalten und Zeilen angeordnete Räume für die Aufzucht von Larven zu verwenden. Eine regelmäßige bzw. gleichmäßige Anordnung von Räumen ist bevorzugt, um eine einfache Handhabung zur Befüllung der Räume zu erreichen. Mehrere Räume können im Wesentlichen gleiche Größe aufweisen. Es kann vorgesehen sein, dass mehrere im Wesentlichen gleich große Räume übereinander in einem Regal ausgebildet sind.

Das Regalbediengerät und der Austragkopf können auch zwanglos kombiniert werden, um zu einer vorteilhaften Ausführungsform der Erfindung zu gelangen. Mittels eines an eine Regalfachanordnung angepassten Austragkopfes für ein Regalbediengerät ist ein synergistischer Effekt zur Lösung der Aufgabe erzielbar.

Bei dem Austragkopf können die mehrere Auslässe (zumindest teilweise gleichzeitig) in ein Regalfach gerichtet werden. Es kann auch vorgesehen sein, dass unterschiedliche Auslässe in unterschiedliche Regalfächer gerichtet werden können.

Die Auslässe können einzeln ansteuerbare Ventile aufweisen. Die Auslässe können endseitig an einem rohrförmigen Ende eines Kanals vorgesehen sein. So kann mittels einer Zuführung, die von einem Vorrat Insektenlarven, Futter und/oder Wasser erhalten kann, der Austragkopf mit dem Fördergut beschickt werden. Am Austragkopf kann ein Verteiler für die Auslässe bzw. rohrförmigen Enden vorgesehen sein, so dass die Zuführung in mehrere Zuführungen "aufgespalten wird", um das Fördergut den einzelnen Regalfächern zuzuführen. Mehrere Auslässe können mittels einer Zuführung beschickt werden. Insbesondere können Gruppen von Auslässen gebildet werden. Eine Gruppe kann beispielsweise übereinander angeordnete Auslässe aufweisen, so dass die übereinander angeordneten Auslässe von einer Zuführung beschickt werden können. Die Zuführung zum Austragkopf kann insbesondere in Form eines Schlauchs oder Rohrs ausgestaltet sein.

Die Zuführung kann einen Rotationsverteiler aufweisen, der ein rundes Verteilergehäuse mit einer Mehrzahl von Öffnungen umfassen kann. Die Mehrzahl der Öffnungen kann in einem Ring auf der Unterseite angeordnet sein. In dem Verteilergehäuse kann ein rotierendes Rohr angeordnet sein, dessen Auslassöffnung sich während einer Verwendung über die Öffnungen bewegen kann. An den Öffnungen können unter Druckluft stehende Güter bzw. Insektenlarven, Futter und/oder Wasser bereitgestellt werden. Mittels des Rotationsverteilers kann ein Gemisch erzeugt und dieses Gemisch dem Austragkopf zugeführt werden. Ein Rotationsverteiler bietet den Vorteil, dass keine engen Radien vorhanden sein müssen, an denen die Insekten anprallen können, wodurch der Transport für die Insekten schonender sein kann und sich die Ausbeute erhöhen kann. Ein weiterer Vorteil liegt darin, dass in dem einen rotierenden Rohr keine Futter- oder Insektenreste verbleiben.

Der Austragkopf kann zwei oder mehr nebeneinander angeordnete Auslässe, die einzeln ansteuerbar sind, aufweisen. Die Auslässe können endseitig an, insbesondere metallenen, rohrartigen Enden vorgesehen sein, die jedes ein Steuerventil aufweisen können. Die Auslässe können relativ zum Austragkopf verfahrbar sein. Der Auslass kann insbesondere in der Achse des rohrartigen Endes verfahrbar sein, um insbesondere in einen Raum einfahren zu können. Die Verfahrbarkeit des Endes bzw. des Auslasses kann im Bereich von einem Meter bis zu wenigen Zentimetern liegen. In einer Ausführungsform kann ein Ende bzw. Auslass um 10 cm bis 30 cm, insbesondere 15 cm bis 25 cm, ganz bevorzugt 20 cm verfahren werden.

Der Austragkopf kann in Gänze in Richtung der Längserstreckung eines Regals und in der Höhe des Regals bewegt werden. Zudem kann vorgesehen sein, dass der Austragkopf in Gänze in Richtung auf ein Regalfach bewegt werden, um einen oder mehrere Auslässe in das Regalfach zu bewegen. Es kann auch vorgesehen sein, dass ein oder mehrere Auslässe am Austragkopf unabhängig voneinander angesteuert werden können, um in Richtung eines oder mehrerer Regalfächer bewegt werden zu können.

In einer bevorzugten Ausführungsform kann der Austragkopf 12 einzeln ansteuerbare Auslässe aufweisen. Die 12 Auslässe können in zwei Gruppen von je sechs Auslässen übereinander angeordnet sein, so dass sich eine zeilen- und spaltenförmige Anordnung ergeben kann, bei der zwei Spalten und sechs Zeilen vorliegen können.

Mittels mehrerer Spalten der Auslässe lässt sich insbesondere mehr als ein Regalfach pro Zeile beschicken. In einer bevorzugten Ausführungsform können zwei, drei vier, fünf oder mehr nebeneinander angeordnete Regalfächer zumindest teilweise gleichzeitig beschickt werden. Es ist auch eine Gestaltung mit einem Kompromiss aus Größe des Austragkopfs (zusammen mit den Auslässen) und Zeit der Beschickung aller Regalfächer des Regals möglich. Je mehr Auslässe vorgesehen sind, desto weniger muss der Austragkopf verfahren werden, um weitere Regalfächer anzusteuern. Mit der Anzahl der anzusteuernden bzw. zu beschickenden Regalfächer steigt die Größe bzw. Ausdehnung des Austragkopfes. In einer besonders bevorzugten Ausführungsform können zwei oder drei, insbesondere zwei nebeneinander angeordnete Regalfächer, beschickt werden. Es ist möglich, dass die beiden Regalfächer direkt nebeneinander liegen; es kann aber auch vorgesehen sein, dass zwei Regalfächer beschickt werden können, die nicht direkt nebeneinander liegen. Durch ein Verfahren des Austragkopfes längs der Regalfächer können die einzelnen Regalfächer nach und nach beschickt werden.

In einer bevorzugten Ausführungsform sind die Regalfächer in einem Regal angeordnet. Die Regalfächer können matrixförmig angeordnet sein. In einer besonders bevorzugten Ausführungsform entspricht die Anzahl der Auslässe am Austragkopf der Anzahl der Regalfächer im Regal in der Höhe, so dass die Regalfächer in einer Spalte des Regals zumindest teilweise gleichzeitig von dem Austragkopf beschickt werden können. Es ist auch möglich, dass die Anzahl der Regalfächer in der Höhe einem Vielfachen der Anzahl der Auslässe, die am Austragkopf übereinander angeordnet sind, entspricht, so dass der Austragkopf eine Anzahl von Positionen in der Höhe verfahren werden muss, die dem Vielfachen entspricht, um alle Regalfächer zu beschicken. Wird der Austragkopf entlang des Regals verfahren, so können die Regalfächer neben der "befüllten" Spalte in weiteren Spalten befüllt werden.

Es kann vorgesehen sein, dass die nebeneinander angeordneten Auslässe zur Zuführung unterschiedlicher Substanzen/Material vorgesehen sind. Beispielsweise kann, nachdem das Futter mittels eines der nebeneinander angeordneten Auslässe eingefüllt wurde, der Austragkopf die Position wechseln und kann mit einem oder dem anderen der nebeneinander angeordneten Auslässe Insektenlarven oder ein Futter/Insektenlarvengemisch in das Regalfach einfüllen, in das zuvor Futter eingebracht wurde.

Die Regalfächer können eine oder mehrere Klappen aufweisen, in die die Enden mit den Auslässen eintauchen können. Die Klappen können selbstverschließend ausgestaltet sein. Beispielsweis kann vorgesehen sein, dass ein Ende in die Klappe und damit in das Regalfach eintauchen kann und beim Herausziehen des Endes die Klappe wieder schließt. Hierdurch kann zumindest an der Zuführung ein geschlossenes Regalfach vorgesehen sein, bei dem verhindert werden kann, dass die Insektenlarven aus dem Regalfach fallen oder wandern.

Es kann eine automatische Zuführung von Insekten/Insektenlarven, Futter und/oder Wasser in die Regalfächer erreicht werden, bei der gezielt gewünschte Mengen von Insekten/Insektenlarven und Futter in die Regalfächer eingebracht werden können. Beispielsweise kann ein Durchfluss berechnet werden bzw. angegeben sein, anhand dessen die Menge an Insektenlarven, Futter und/oder Wasser in Abhängigkeit von der Zeit bestimmt werden kann. Es ist in einer bevorzugten Ausführungsform möglich, eine vorbestimmte Insektenlarvenmenge in das Regalfach mit einer vorbestimmten Futtermenge in dem gleichen Regalfach anzuordnen.

Die Zuführung von Wasser in das Regalfach ist insbesondere nach Ablauf der Mastzeit der Insektenlarven gewünscht, um die Insektenlarven aus dem Regalfach zu entfernen und die Insektenlarven weiteren Verarbeitungsschritten zuzuführen. Zu diesem Zweck kann den Regalfächern mittels derselben Auslässe, die schon zur Fütterung genutzt wurden, Wasser zugeführt werden. Hierzu können die Enden durch die Klappe in die Regalfächer gesteckt werden und das Insektenlarven-Kot-Futter-Gemisch des Regalfachs mittels des Wassers durch eine, insbesondere rückseitig angebrachte Klappe, aus dem Regalfach ausgespült werden.

Sofern an dem Austragkopf an zwei gegenüberliegenden Seiten Auslässe vorgesehen sind, so können zwei gegenüberliegende Regale mit ihren Regalfächern mittels des Austragkopf mit Insektenlarven, Futter und/oder Wasser befüllt werden. Es kann so eine hohe Effizienz der Befüllung und der Handhabung bei der Insektenzucht erreicht werden.

Der Austragkopf kann Teil eines Regalbediengeräts sein bzw. an einem Regalbediengerät befestigt sein.

Mittels einer Bewegung des Austragkopfs können mehrere Regalfächer gleichzeitig angefahren und auch beschickt werden. So kann eine blockweise Beschickung von beispielsweise zwei Bioreaktoren ermöglicht werden. Die Zuführung in die einzelnen Regalfächer kann vereinfacht sein, indem die Zuführung erst am Austragkopf einen Verteiler bzw. eine Aufspaltung in mehrere Zuführungen für die Regalfächer erfahren muss.

Eine Automatisierung der Beschickung der Regalfächer ist möglich.

Es kann eine Druckluftquelle vorgesehen sein, die mit dem Austragkopf verbunden werden kann. Der Austragkopf kann mittels der Druckluftquelle mit Druckluft beaufschlagt werden, um den im Austragkopf befindlichen Inhalt aus dem Austragkopf auszublasen. Hierdurch kann eine Restentleerung des Austragkopfes erfolgen, wenn beispielsweise die Zuführung von Insekten, Futter und/oder Wasser unterbrochen und der Austragkopf mit geöffnetem Auslass oder geöffneten Auslässen mit Druckluft beaufschlagt wird. Es kann verhindert werden, dass Insekten oder Futterreste im Austragkopf verbleiben und unkontrolliert ausfließen können. Die Hygiene kann verbessert und einer Verkeimung des Austragkopfes kann vorgebeugt werden.

Der Begriff "Druckluftquelle" umfasst eine Vorrichtung zur Erzeugung von Druckluft, d.h. komprimierter Luft. Die Luft kann zur Verbesserung einer Restentleerung bzw. Reinigung des Austragkopfes temperiert bzw. aufgeheizt sein. Es ist ferner möglich, der Druckluft einen Fluidnebel, insbesondere einen Wassernebel, hinzuzufügen, um die Restentleerung bzw. Reinigung zu erhöhen.

Die Druckluftquelle kann unmittelbar mit dem Austragkopf verbunden sein, indem die Druckluftquelle direkt am Austragkopf angeordnet ist. Es kann aber auch vorgesehen sein, dass die Druckluftquelle vom Austragkopf beabstandet ist und eine Zuführung zwischen Druckluftquelle und Austragkopf in Form eines Kanals, Durchgangs, Rohrs oder dergleichen vorliegt.

Ferner kann die Druckluft über eine Druckluftdüse mit dem Austragkopf verbunden sein, die in Bezug auf die Anordnung im oder am Austragkopf flexibel oder fest ausgerichtet sein kann. Eine derartige Anordnung ermöglicht die gezielte Zuführung der Druckluft an eine gewünschte Stelle und daher eine effiziente Restentleerung des Austragkopfes. Beispielsweise kann die Druckluftdüse bei einer Beaufschlagung mit Druckluft einer vorbestimmten Stelle Druckluft zuführen, wobei die Druckluftdüse insbesondere fest im oder am Austragkopf angeordnet sein kann. Bei einer flexiblen Anordnung einer Druckluftdüse kann mittels einer Bewegung der Druckluftdüse relativ zum Austragkopf ein größerer Bereich überstrichen werden; die Bewegung der Druckluftdüse kann durch die Beaufschlagung der Druckluftdüse mit Druckluft induziert sein.

In den Ansprüchen und der Beschreibung werden Merkmale hinsichtlich unterschiedlicher Aspekte der Erfindung genannt, die eine Vorrichtung/Einrichtung, insbesondere einen Austragkopf und eine Aufzuchtstation, eine Verwendung und ein Verfahren betreffen. Die einzelnen Aspekte ergänzen mit ihren genannten Merkmalen einander hinsichtlich der Ausgestaltung der Merkmale für jeden der anderen Aspekte, sodass insbesondere Merkmale eines Verfahrens auch für die Beschreibung des jeweils anderen Aspekts mittels Vorrichtungsmerkmalen bzw. Verwendungsmerkmalen gelten. Sind Merkmale hinsichtlich eines Aspekts beschrieben, so ist diese Beschreibung als auch als Beschreibung für ein Merkmal eines anderen Aspekts zu verstehen.

## Patentansprüche

1. Einrichtung zur Aufzucht von Insekten,
wobei die Einrichtung ein Regal mit mehreren Regalfächern und ein Regalbediengerät aufweist,
wobei am Regalbediengerät ein Austragkopf angeordnet ist,
wobei der Austragkopf mehrere nebeneinander und/oder übereinander angeordnete Auslässe, aufweist, die zur wahlweisen Zuführung von Insektenlarven, Futter oder Wasser, in ein Regalfach, insbesondere eine Wanne, verfahrbar sind, um in ein Regalfach Insektenlarven, Futter oder Wasser abzugeben, wobei der Austragkopf mit einer Zuführung verbunden ist, mittels derer der Austragkopf mit dem jeweiligen Gut in Form von Insektenlarven, Futter und/oder Wasser beschickt werden kann;
wobei das Regal und der Austragkopf mit den Auslässen dazu ausgestaltet sind, zumindest teilweise gleichzeitig mehreren übereinander und/oder nebeneinander angeordneten Regalfächern Insektenlarven, Futter oder Wasser zuzuführen,
**dadurch gekennzeichnet, dass**
das Regalbediengerät dazu eingerichtet ist, entlang der Längserstreckung und der Höhe des Regals zu verfahren, wobei das Regalbediengerät ein mittels des Regalbediengeräts verfahrbares Lastaufnahmemittel aufweist, das den Austragkopf aufweist, so dass der Austragkopf entlang der Längserstreckung und der Höhe des Regals verfahrbar ist.

2. Einrichtung nach Anspruch 1, wobei eine Druckluftquelle vorhanden ist, die mit dem Austragkopf verbunden werden kann und dazu ausgestaltet ist, den Austragkopf mit Druckluft zu beaufschlagen, um im Austragkopf befindlichen Inhalt aus dem Austragkopf auszublasen.

3. Einrichtung nach Anspruch 1 oder 2, wobei ein Rotationsverteiler zum Mischen von Insektenlarven, Futter und/oder Wasser in der Zuführung zum Austragkopf vorhanden ist, wobei der Rotationsverteiler ein rundes Verteilergehäuse mit einer Mehrzahl von Öffnungen umfasst, wobei in dem Verteilergehäuse ein rotierendes Rohr mit einer Auslassöffnung angeordnet ist, wobei die Auslassöffnung sich während einer Verwendung der Einrichtung über die Öffnungen bewegen kann, so dass an den Öffnungen unter Druckluft stehende Güter bzw. Insektenlarven, Futter und/oder Wasser bereitgestellt werden können.

4. Einrichtung nach Anspruch 3, wobei der Austragkopf in Spalten und Zeilen angeordnete Auslässe aufweist.

5. Einrichtung nach Anspruch 3 oder 4, wobei der Austragkopf in entgegengesetzte Richtungen weisende Auslässe aufweist, um insbesondere in zwei gegenüberliegende Regalfächer Insektenlarven, Futter oder Wasser zumindest teilweise gleichzeitig abzugeben.

6. Einrichtung nach einem der Ansprüche 3 bis 5, wobei ein Auslass relativ zum Austragkopf verfahrbar ist.

7. Einrichtung nach einem der Ansprüche 3 bis 6, wobei ein Auslass an einem Rohr bzw. rohrartigen Ende vorgesehen ist, und sich Rohre bzw. Enden von dem Austragkopf parallel zueinander weg erstrecken.

8. Einrichtung nach Anspruch 7, wobei das Rohr oder Ende von dem Austragkopf in Richtung eines Regalfachs verfahren werden kann.

9. Einrichtung nach einem der Ansprüche 3 bis 8, wobei mehrere Auslässe, insbesondere die untereinander angeordneten Auslässe, mit der gleichen Zuführung zum Austragkopf verbunden sind.

10. Einrichtung nach einem der Ansprüche 3 bis 9, wobei mehrere Auslässe, insbesondere die nebeneinander angeordneten Auslässe, mit unterschiedlichen Zuführungen zum Zuführen unterschiedlichen Guts verbunden sind.

11. Verfahren zum Zuführen von Insektenlarven, Futter und/oder Wasser in ein Regalfach einer Insektenaufzuchtstation,
wobei ein Regal bereitgestellt wird, das Regalfächer aufweist;
wobei ein Regalbediengerät bereitgestellt wird, wobei das Regalbediengerät dazu eingerichtet ist, entlang der Längserstreckung und der Höhe des Regals zu verfahren;
wobei am Regalbediengerät ein Austragkopf bereitgestellt wird,
wobei der Austragkopf mehrere nebeneinander und/oder übereinander angeordnete Auslässe aufweist, die zur wahlweisen Zuführung von Insektenlarven, Futter oder Wasser, in ein Regalfach, insbesondere eine Wanne, verfahrbar sind, um in ein Regalfach Insektenlarven, Futter oder Wasser abzugeben, wobei der Austragkopf mit einer Zuführung verbunden ist, mittels derer der Austragkopf mit dem jeweiligen Gut in Form von Insektenlarven, Futter und/oder Wasser beschickt werden kann,
wobei das Regal und der Austragkopf mit den Auslässen derart ausgestaltet sind, um zumindest teilweise gleichzeitig mehreren übereinander und/oder nebeneinander angeordneten Regalfächern Insektenlarven, Futter oder Wasser zuzuführen;
wobei das Regalbediengerät ein mittels des Regalbediengeräts verfahrbares Lastaufnahmemittel aufweist, das den Austragkopf aufweist, so dass der Austragkopf entlang der Längserstreckung und der Höhe des Regals verfahrbar ist; und
wobei den Regalfächern wahlweise Insektenlarven, Futter oder Wasser mittels des Austragkopfs zugeführt werden.

12. Verfahren nach Anspruch 11, wobei die Auslässe zur Zuführung unterschiedlicher Substanzen vorgesehen sind.

## Claims

1. Device for rearing insects,
the device comprising a shelving unit which has a plurality of shelf compartments and comprising a storage and retrieval machine,
a discharge head being arranged on the storage and retrieval machine,
the discharge head having a plurality of outlets which are arranged next to one another and/or one above the other and which, for selectively supplying insect larvae, feed or water, can be moved into a shelf compartment, in particular a trough, in order to dispense insect larvae, feed or water into a shelf compartment, the discharge head being connected to a supply, by means of which the discharge head can be supplied with the relevant material in the form of insect larvae, feed and/or water;
the shelving unit and the discharge head which has the outlets being designed to supply insect larvae, feed or water at least partially simultaneously to a plurality of shelf compartments arranged one above the other and/or next to one another,
**characterized in that**
the storage and retrieval machine is designed to move along the longitudinal extent and the height of the shelving unit, the storage and retrieval machine having a load handling means which can be moved by means of the storage and retrieval machine and which has the discharge head, so that the discharge head can be moved along the longitudinal extent and the height of the shelving unit.

2. Device according to claim 1, wherein a compressed air source is provided which can be connected to the discharge head and is designed to supply the discharge head with compressed air in order to blow contents located in the discharge head out of the discharge head.

3. Device according to claim 1 or claim 2, wherein a rotary distributor for mixing insect larvae, feed and/or water is provided in the supply to the discharge head, wherein the rotary distributor comprises a round distributor housing having a plurality of openings, wherein a rotating tube having an outlet opening is arranged in the distributor housing, wherein the outlet opening can move over the openings during use of the device, so that materials, more specifically insect larvae, feed and/or water, under compressed air can be provided at the openings.

4. Device according to claim 3, wherein the discharge head has outlets arranged in columns and rows.

5. Device according to claim 3 or claim 4, wherein the discharge head has outlets pointing in opposite directions in order to dispense insect larvae, feed or water at least partially simultaneously, in particular into two opposite shelf compartments.

6. Device according to any of claims 3 to 5, wherein an outlet can be moved relative to the discharge head.

7. Device according to any of claims 3 to 6, wherein an outlet is provided at a tube or tube-like end, and tubes or ends extend away from the discharge head in parallel with one another.

8. Device according to claim 7, wherein the tube or end can be moved by the discharge head in the direction of a shelf compartment.

9. Device according to any of claims 3 to 8, wherein a plurality of outlets, in particular the outlets arranged one below the other, are connected to the same supply leading to the discharge head.

10. Device according to any of claims 3 to 9, wherein a plurality of outlets, in particular the outlets arranged next to one another, are connected to different supplies for supplying different materials.

11. Method for supplying insect larvae, feed and/or water into a shelf compartment of an insect rearing station,
wherein a shelving unit having shelf compartments is provided,
wherein a storage and retrieval machine is provided, wherein the storage and retrieval machine is designed to move along the longitudinal extent and the height of the shelving unit;
wherein a discharge head is provided on the storage and retrieval machine,
wherein the discharge head has a plurality of outlets which are arranged next to one another and/or one above the other and which, for selectively supplying insect larvae, feed or water, can be moved into a shelf compartment, in particular a trough, in order to dispense insect larvae, feed or water into a shelf compartment, wherein the discharge head is connected to a supply, by means of which the discharge head can be supplied with the relevant material in the form of insect larvae, feed and/or water,
wherein the shelving unit and the discharge head which has the outlets are designed to supply insect larvae, feed or water at least partially simultaneously to a plurality of shelf compartments arranged one above the other and/or next to one another;
wherein the storage and retrieval machine has a load handling means which can be moved by means of the storage and retrieval machine and which has the discharge head, so that the discharge head can be moved along the longitudinal extent and the height of the shelving unit; and
wherein insect larvae, feed or water are selectively supplied to the shelf compartments by means of the discharge head.

12. Method according to claim 11, wherein the outlets are provided for supplying different substances.

## Revendications

1. Installation permettant l'élevage d'insectes,
dans laquelle l'installation présente une étagère comportant plusieurs compartiments d'étagère et un transstockeur,
dans laquelle une tête de déchargement est disposée sur le transstockeur,
dans laquelle la tête de déchargement présente plusieurs sorties disposées les unes à côté des autres et/ou les unes au-dessus des autres, qui peuvent être déplacées dans un compartiment d'étagère, en particulier une cuve, pour l'apport au choix de larves d'insectes, de nourriture pour animaux ou d'eau, pour délivrer des larves d'insectes, de la nourriture pour animaux ou de l'eau dans un compartiment d'étagère, dans laquelle la tête de déchargement est reliée à un apport à l'aide duquel la tête de déchargement peut être alimentée avec le produit respectif sous la forme de larves d'insectes, de nourriture pour animaux et/ou d'eau ;
dans laquelle l'étagère et la tête de déchargement comportant les sorties sont réalisées pour apporter au moins partiellement simultanément des larves d'insectes, de la nourriture pour animaux ou de l'eau à plusieurs compartiments d'étagère disposés les uns au-dessus des autres et/ou les uns à côté des autres,
**caractérisée en ce que**
le transstockeur est conçu pour se déplacer le long de l'extension longitudinale et de la hauteur de l'étagère, dans laquelle le transstockeur présente un moyen de réception de charge pouvant être déplacé à l'aide du transstockeur, qui présente la tête de déchargement, en sorte que la tête de déchargement peut être déplacée le long de l'extension longitudinale et de la hauteur de l'étagère.

2. Installation selon la revendication 1, dans laquelle une source d'air comprimé est disponible, qui peut être reliée à la tête de déchargement et qui est réalisée pour alimenter la tête de déchargement en air comprimé pour souffler le contenu se trouvant dans la tête de déchargement hors de la tête de déchargement.

3. Installation selon la revendication 1 ou 2, dans laquelle un distributeur rotatif permettant le mélange de larves d'insectes, de nourriture pour animaux et/ou d'eau est disponible dans l'apport vers la tête de déchargement, dans laquelle le distributeur rotatif comprend un boîtier de distributeur circulaire comportant une pluralité d'ouvertures, dans laquelle un tube rotatif comportant une ouverture de sortie est disposé dans le boîtier de distributeur, dans laquelle l'ouverture de sortie peut se déplacer sur les ouvertures pendant une utilisation de l'installation, en sorte que des produits ou des larves d'insectes, de la nourriture pour animaux et/ou de l'eau sous pression peuvent être fournies au niveau des ouvertures.

4. Installation selon la revendication 3, dans laquelle la tête de déchargement présente des sorties disposées en colonnes et en lignes.

5. Installation selon la revendication 3 ou 4, dans laquelle la tête de déchargement présente des sorties orientées dans des directions opposées, en particulier pour délivrer au moins partiellement simultanément des larves d'insectes, de la nourriture pour animaux ou de l'eau dans deux compartiments d'étagère opposés.

6. Installation selon l'une des revendications 3 à 5, dans laquelle une sortie peut être déplacée par rapport à la tête de déchargement.

7. Installation selon l'une des revendications 3 à 6, dans laquelle une sortie est prévue sur un tube ou une extrémité tubulaire, et des tubes ou des extrémités s'étendent parallèlement les uns aux autres à partir de la tête de déchargement.

8. Installation selon la revendication 7, dans laquelle le tube ou l'extrémité peut être déplacé(e) par la tête de déchargement en direction d'un compartiment d'étagère.

9. Installation selon l'une des revendications 3 à 8, dans laquelle plusieurs sorties, en particulier les sorties disposées les unes sous les autres, sont reliées au même apport de la tête de déchargement.

10. Installation selon l'une des revendications 3 à 9, dans laquelle plusieurs sorties, en particulier les sorties disposées les unes à côté des autres, sont reliées à différents apports permettant l'apport de différents produits.

11. Procédé permettant l'apport de larves d'insectes, de nourriture pour animaux et/ou d'eau à un compartiment d'étagère d'une station d'élevage d'insectes,
dans lequel une étagère est fournie, laquelle présente des compartiments d'étagère ;
dans lequel un transstockeur est fourni, dans lequel le transstockeur est conçu pour se déplacer le long de l'étendue longitudinale et de la hauteur de l'étagère ;
dans lequel une tête de déchargement est fournie sur le transstockeur,
dans lequel la tête de déchargement présente plusieurs sorties disposées les unes à côté des autres et/ou les unes au-dessus des autres, qui peuvent être déplacées dans un compartiment d'étagère, en particulier une cuve, pour l'apport au choix de larves d'insectes, de nourriture pour animaux ou d'eau, pour délivrer des larves d'insectes, de la nourriture pour animaux ou de l'eau dans un compartiment d'étagère, dans lequel la tête de déchargement est reliée à un apport à l'aide duquel la tête de déchargement peut être alimentée avec le produit respectif sous la forme de larves d'insectes, de nourriture pour animaux et/ou d'eau,
dans lequel l'étagère et la tête de déchargement comportant les sorties sont réalisées de manière à apporter au moins partiellement simultanément des larves d'insectes, de la nourriture pour animaux ou de l'eau à plusieurs compartiments d'étagère disposés les uns au-dessus des autres et/ou les uns à côté des autres ;
dans lequel le transstockeur présente un moyen de réception de charge pouvant être déplacé du transstockeur, qui présente la tête de déchargement, en sorte que la tête de déchargement peut être déplacée le long de l'extension longitudinale et de la hauteur de l'étagère ; et
dans lequel des larves d'insectes, de la nourriture pour animaux ou de l'eau sont apportés au choix aux compartiments d'étagère à l'aide de la tête de déchargement.

12. Procédé selon la revendication 11, dans lequel les sorties sont prévues pour l'apport de différentes substances.
